(19) Europäisches Patentamt
European Patent Office
Office européen des brevets

(11) **EP 2 039 371 B1**

(12) # EUROPEAN PATENT SPECIFICATION

(45) Date of publication and mention
of the grant of the patent:
**09.11.2011 Bulletin 2011/45**

(51) Int Cl.:
***A61L 9/12*** *(2006.01)*

(21) Application number: **08253096.5**

(22) Date of filing: **23.09.2008**

(54) **Fragrance emitting patch**

Duftspendepflaster

Timbre émettant des fragrances

(84) Designated Contracting States:
**DE FR GB IT**

(30) Priority: **24.09.2007 US 974659 P**

(43) Date of publication of application:
**25.03.2009 Bulletin 2009/13**

(73) Proprietor: **McNeil-PPC, Inc.
Skillman, NJ 08558 (US)**

(72) Inventors:
• **Burrow, Ricky Ray
Doylestown
PA 18901 (US)**
• **Gannon, Elaine M.
Hoboken
NJ 07030 (US)**

• **Luizzi, Joseph M.
Newtown
PA 18940 (US)**
• **Mavinkurve, Pramod
Princeton
NJ 08540 (US)**
• **Moscherosch, Michael H.
Doylestown
PA 19801 (US)**

(74) Representative: **Kirsch, Susan Edith et al
Carpmaels & Ransford
One Southampton Row
London
WC1B 5HA (GB)**

(56) References cited:
**EP-A- 1 854 442      WO-A-2004/110401
GB-A- 1 461 730      US-A- 4 880 690
US-A- 5 399 404      US-A1- 2007 031 463**

**Description**

The present invention relates to a fragrance emitting patch that a user can attach to the body or an article of clothing, and more particularly to a fragrance emitting patch including at least one layer including a fragrance and an adhesive applied to the patch for selectively securing the patch to the body or an article of clothing.

## BACKGROUND OF THE INVENTION

Fragrance emitting devices are generally used to deliver a pleasing scent to the user. These devices have been used in the past to mask undesirable odors and can also be functionalized with an odor-controlling agent. The prior art discloses fragrance emitting patches that purport to deliver fragrance when a user attaches the patch onto their body or an article of clothing. These patches generally include one more or more layers of material, at least one of which is provided with a fragrance. Fragrance emitting patches generally include a positioning adhesive applied to an external surface of the patch for selectively adhering the patch onto the user's skin or article of clothing. Patches with multiple layers may also include a construction adhesive used to affix the layers of the patch to one another.

US 5399404 and US 2006/0251609 disclose fragrance emitting patches.

The inventors have discovered that many fragrances used in known fragrance emitting patches will migrate into the adhesive components of the patch and undesirably interact with the adhesive by altering its chemical composition. In particular, the inventors have discovered the aromatic components of many fragrances tend to plasticize the end blocks of standard hot melt adhesives. The inventors have discovered that this interaction causes the adhesives to perform poorly by reducing the cohesiveness and internal strength of standard construction and positioning adhesives. Specifically, the inventors have discovered that the interaction between the fragrance and positioning adhesive may cause the patch to detach from the surface to which it is applied and in a multilayer construction the interaction of the fragrance with the construction adhesive may cause the undesirable delamination of the layers of the patch.

The inventors have further discovered that in order for a fragrance emitting patch to provide the desired intensity of scent, and in order for the scent to last for a sufficient period of time during use, the fragrance must be applied to the relevant layer of the patch in a relatively high add on amount. However, the inventors have discovered that the use of a high add on amount of fragrance exacerbates the degradation of the adhesive described above.

In view of the foregoing, the present invention provides a fragrance emitting patch that has the ability to incorporate high levels of fragrance without sacrificing the functionality of the construction and positioning adhesives used within the patch.

## SUMMARY OF THE INVENTION

In view of the foregoing, the present invention provides, according to a first aspect of the invention, a fragrance emitting patch including a primary porous layer having a top and a bottom surface, a secondary porous layer having a top and a bottom surface, a construction adhesive arranged between the primary layer and the secondary layer for securing the primary and secondary layers to one another, wherein the primary layer is provided with a fragrance in an amount within the range of 3 g/m$^2$ to 15 g/m$^2$, and wherein an absolute difference of a Hildebrand solubility parameter of the construction adhesive and a Hildebrand solubility parameter of the fragrance is greater than 1.5.

The present invention provides, according to a second aspect of the invention, a fragrance emitting patch including a primary porous layer having a top and a bottom surface, a secondary porous layer having a top and a bottom surface, a positioning adhesive deposited on the bottom surface of the secondary layer, a construction adhesive arranged between the primary layer and the secondary layer for securing the primary and secondary layers to one another, wherein at least one of the first and second layers is provided with a fragrance in an amount within the range of 3 g/m$^2$ to 15 g/m$^2$, wherein an absolute difference of a Hildebrand solubility parameter of the construction adhesive and a Hildebrand solubility parameter of the fragrance is greater than 1.5.

## BRIEF DESCRIPTION OF THE DRAWINGS

FIG. 1 is a schematic view of a fragrance emitting patch in accordance with the invention adhered to an undergarment;

FIG. 2 is a bottom plan view of a fragrance emitting patch in accordance with the invention with the removable backing layer thereof partially torn away to reveal the positioning adhesive thereunder;

FIG. 3 is a cross sectional view of the patch shown in FIG. 2 taken along line 3-3 thereof;

FIG. 4 is a top plan view of a fragrance emitting patch in accordance with another embodiment of the invention;

FIG. 5 is a cross sectional view of the patch shown in FIG. 4 taken along line 5-5 thereof;

FIG. 6 is a top plan view of a fragrance emitting patch in accordance with yet another embodiment of the invention;
FIG. 7 is a cross sectional view of the patch shown in FIG. 6 taken along line 7-7 thereof.

## DETAILED DESCRIPTION OF THE INVENTION

As used herein, the term "construction adhesive" refers to any adhesive that is used to join two layers of material to one another.

As used herein, the term "positioning adhesive" refers to any adhesive that is used to removably attach a fragrance emitting patch to a user's skin or clothing.

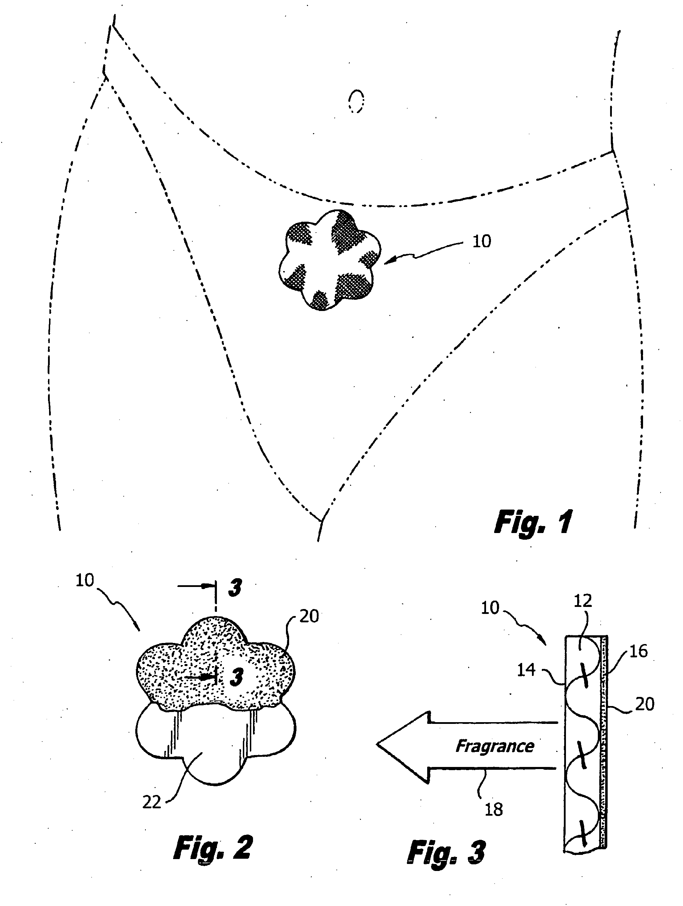

In one embodiment of the invention, as shown in Fig. 1, the fragrance emitting patch according to the present invention is intended to be applied to a user's undergarment, such as a woman's panty, during use, to thereby provide a fresh scent to undergarment. The inventors have found that in order to achieve long lasting fragrance levels that can be detected from the perennial region of the user to the nose, a high amount of fragrance must be incorporated into the fragrance emitting patch. The inventors have found that users can detect a product including a fragrance applied to one of the layers of the patch in an add on amount of greater than 3 gsm ($g/m^2$). In one embodiment of the invention, a fragrance is applied to at least one of the layers of the fragrance emitting patch in an amount of between about 3 gsm and about 15 gsm.

The inventors have discovered that purposely selecting fragrances that are substantially insoluable in the adhesive compounds used in the fragrance emitting patch minimizes the undesireable reaction between these components. The Hildebrand solubility parameter is used often in chemistry to predict when two solutions are soluble in one another. According to the theory proposed by Dr. Joel Hildebrand, two solutions will be soluble when the Hildebrand solubility parameter is equal, and insoluble when the Hildebrand solubility parameter is not equal. The difference between the two values is roughly related to the extent of insolubility between the two solutions. The Hildebrand solubility parameter ($\delta(SI)$) is derived from the heat of vaporization ($\Delta H$), the universal gas constant (R), the temperature (T), and the molar volume of the solution ($V_m$), and is calculated using the following formula:

$$\delta(SI) = [(\Delta H - RT) / V_m]^{1/2}$$

The resulting value is a property of a particular solution at a given temperature. In the international system of units (SI), the universal gas constant (R) is approximately 8.314 $J \cdot K^{-1} \cdot mol^{-1}$. The Hildebrand solubility parameter has the units of $MPa^{1/2}$.

The Hildebrand solubility parameter of common adhesives and fragrances is provided in TABLE 1 below:

**TABLE 1**

| Classification | Solution | $\delta(SI)$ |
|---|---|---|
| Common Adhesives | Sytrenic Block Copolymers and Tackifying Resins | 14.4-18.6 |
| | Polyethylene, EVA | 17-18.6 |
| | Polypropylene Polymers | 17.2-19.2 |
| Common Fragrances | Pine Oil | 17.6 |
| | d-Limonene | 16.5 |
| | Vanillin | 24.7 |
| | Eugenol | 22.2 |
| | Citral | 18.7 |
| | Carvone | 18.7 |
| | Jasmone | 18.4 |

According to the present invention, the adhesive(s) and fragrance(s) employed in the fragrance emitting patch have a solubility parameter absolute value difference of greater than 1.5, preferably greater than 3.0, and most preferably greater than 5.0. This relationship can be expressed by the follow equation:

$$|\delta_a - \delta_f| > 1.5;$$

where

$\delta_a$ = Hildebrand solubility parameter of the adhesive, and

$\delta_f$ = Hildebrand solubility parameter of the fragrance.

Selection of a fragrance(s) and an adhesive(s) 20 satisfy the above equation insures that fragrance does not adversely interact with the ahesive and thereby compromise the same. This insures that, even at high fragrance add on levels, the fragrance emitting patch will securly adhere to the surface to which it is applied and will not delaminate.

## DESCRIPTION OF THE PREFERRED EMBODIMENTS

Fig. 1 illustrates an example of a fragrance emitting patch 10 according to the present invention, the patch 10 is adhered to the surface of a woman's undergarment to provide a fresh scent thereto. Referring to Figs. 2 and 3, the patch 10 includes, according to a first embodiment of the invention, a porous primary layer 12 having a top surface 14 and a bottom surface 16. The primary layer 12 is provided with a fragrance represented by the numeral 18. The fragrance 18 may be applied to a top surface 14 or bottom 16 surface of the primary layer 12 or it may be infused into the primary layer 12 itself. The patch 10 is further provided with a positioning adhesive 20 applied to the bottom surface 16 of the primary layer 12. The positioning adhesive 20 allows a user to selectively apply the patch to a garment of clothing such as an undergarment, or directly to the skin.

The fragrance emitting patch 10 may be optionally provided with a removable backing layer 22, shown in Fig. 2, that is intended to protect the positioning adhesive 20 prior to use of the patch 10. The backing layer 22 may be constructed of a suitable paper and/or polymeric film material. The surface of the backing layer 22 in contact with the positioning adhesive 20 may be provided with a non-stick coating such as silicone to facilitate the removal of the backing layer 22 by the user prior to use.

According to one aspect of the invention, the primary layer 12 is constructed from a porous non-woven web material. The primary layer 12 may be composed of only one type of fiber, such as polyester or polypropylene or it may include a mixture of more than one fiber. The primary layer 12 may be composed of bi-component or conjugate fibers having a low melting point component and a high melting point component. The fibers may be selected from a variety of natural and synthetic materials such as nylon, polyester, rayon (in combination with other fibers), cotton, acrylic fiber and the like and combinations thereof. Preferably, the primary layer 12 has a basis weight in the range of about 10 gsm to about 75 gsm. Bi-component fibers may be made up of a polyester layer and a polyethylene sheath. Using a fusible fabric increases the ease with which the primary layer 12 may be mounted to any underlying layer should such an underlying layer be employed. According to another aspect of the invention the porous primary layer 12 is constructed from a microporous polymeric film material.

According to one aspect of the invention, the porous primary layer 12 is provided with the fragrance 18. The fragrance 18 may be selected from one of the fragrances set forth in Table 1 above or may be selected from other common fragrances known to those of skill in the art. The fragrance 18 may also constitute a complex fragrance, i.e. a fragrance including a mixture of a number of different fragrance components. Typically the solubility parameter $\delta_f$ of such complex fragrance mixtures may be obtained from the commercial manufacturer of such fragrances. The fragrance 18 is preferably provided on or in the primary layer 12 in an amount greater than about 3 gsm ($g/m^2$), preferably between about 3 gsm and about 15 gsm.

According to one aspect of the invention, the bottom surface 16 of the porous primary layer 12 is provided with a positioning adhesive 20. Preferably the positioning adhesive 20 is applied to the bottom surface 16 in an amount between about 8 gsm to about 25 gsm. Suitable positioning adhesive 20 compositions include hot melt adhesives based on block copolymers such as linear or radial co-polymer structures having the formula $(A-B)_x$ wherein block A is a polyvinylarene block, block B is a poly(monoalkenyl) block, and x is an integer greater than or equal to one that denotes the number of polymeric arms. Suitable block A polyvinylarenes include, but are not limited to, polystyrene, polyalpha-methylstyrene, polyvinyltoluene, and combinations thereof. Likewise, suitable Block B poly(monoalkenyl) blocks include, but are not limited to, conjugated diene elastomers, such as polybutadiene, polyisoprene, and hydrogenated elastomers such as ethylene butylenes, ethylene propylene, polyisobutylene, or combinations thereof. Commercial examples of these types of block copolymers include KratonTM elastomers from Kraton Polymers L.P, VectorTM elastomers from Dexco, SIBSTAR polymers from Kaneka USA, and StereonTM from Firestone Tire & Rubber Co. Alternately, suitable acrylic hot melt adhesive polymers such as the ACResin hot melt adhesives from BASF Corp. may also be used. In addition to providing some level of insolubility to the fragrances, these systems can be rendered further insoluble via crosslinking using a UV radiation source.

According to the present invention, the positioning adhesive 20 and the fragrance 18 should be selected such that they have a solubility parameter absolute value difference of greater than 1.5, preferably greater than 3.0 and most preferably greater than 5.0. This relationship can be expressed by the follow equation:

$$|\delta_{pa} - \delta_f| > 1.5;$$

where

$\delta_{pa}$ = Hildebrand solubility parameter of the positioing adhesive, and
$\delta_f$ = Hildebrand solubility parameter of the fragrance.

Selection of a fragrance 18 and a positioining adhesive 20 that satisfy the above equation insures that fragrance 18 does not adversely interact with the positioning ahesive 20 and thereby compromise the same. This insures that, even at high fragrance add on levels, the fragrance emitting patch will securely adhere to the surface to which it is applied.

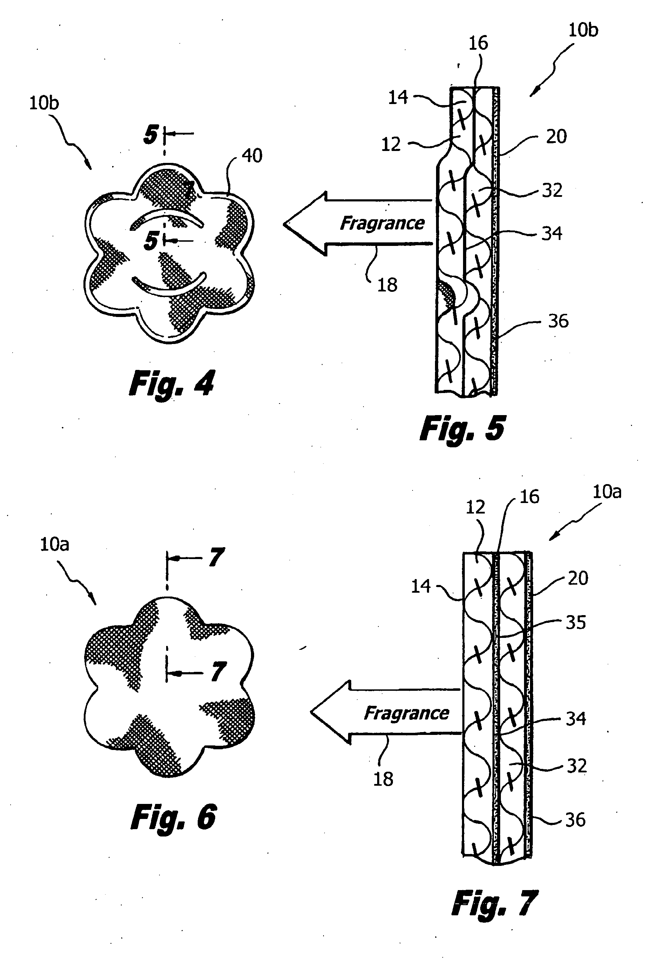

Referring to Figs. 6 and 7, a fragrance emitting patch 10a includes according to another embodiment of the invention, a porous primary layer 12 having a top surface 14 and a bottom surface 16. The primary layer 12 is provided with a fragrance represented by the numeral 18. The fragrance 18 may be applied to a top surface 14 or bottom 16 surface of the primary layer 12 or it may be infused into the primary layer 12 itself. The patch 10 further includes a secondary layer 32 having a top surface 34 and bottom surface 36. The secondary layer 32 is adhered to a bottom surface 16 of the primary layer 12 by a construction adhesive 35 that is arranged between the layers 12 and 32. The bottom surface 36 of the secondary layer 32 is provided with the positioning adhesive 20 that permits a user to selectively apply the patch 10a to a garment of clothing such as an undergarment or directly to the skin.

The construction adhesive 35 is preferably selected from the same group of adhesives as the positioing adhesive 20. Thus suitable construction adhesive 35 compositions include hot melt adhesives based on block copolymers such as linear or radial co-polymer structures having the formula $(A-B)_x$ wherein block A is a polyvinylarene block, block B is a poly(monoalkenyl) block, and x is an integer greater than or equal to one that denotes the number of polymeric arms. Suitable block A polyvinylarenes include, but are not limited to, polystyrene, polyalpha-methylstyrene, polyvinyltoluene, and combinations thereof. Likewise, suitable Block B poly(monoalkenyl) blocks include, but are not limited to, conjugated diene elastomers, such as polybutadiene, polyisoprene, and hydrogenated elastomers such as ethylene butylenes, ethylene propylene, polyisobutylene, or combinations thereof. Commercial examples of these types of block copolymers include KratonTM elastomers from Kraton Polymers L.P, VectorTM elastomers from Dexco, SIBSTAR polymers from Kaneka USA, and StereonTM from Firestone Tire & Rubber Co. Alternately, suitable acrylic hot melt adhesive polymers such as the ACResin hot melt adhesives from BASF Corp. may also be used. In addition to providing some level of insolubility to the fragrances, these systems can be rendered further insoluble via crosslinking using a UV radiation source.

As shown in Fig. 7, the construction adhesive 35 is preferably applied to a bottom surface 16 of the primary layer 12 in an amount between 1 gsm and 25 gsm. According to an aspect of the invention, the construction adhesive 35 and the fragrance 18 are applied in an amount such that a ratio of the amount of construction adhesive 35 employed in the patch 10a to amount of fragrance 18 incorporated into the patch 10a is between about 0.333 to about 1.67. This relationship can be expressed by the following equation:

$$1.67 > A_{ca} / A_f > 0.333,$$

where

$A_{ca}$ = Add on amount of construction adhesive, and
$A_f$ = Add on amount of fragrance.

According to the present invention, the construction adhesive 35 and the fragrance 18 should be selected such that they have a solubility parameter absolute value difference of greater than 1.5, preferably greater than 3.0 and most preferably greater than 5.0. This relationship can be expressed by the follow equation:

$$|\delta_{ca} - \delta_f| > 1.5;$$

where

$\delta_{ca}$ = Hildebrand solubility parameter of the construction adhesive, and
$\delta_f$ = Hildebrand solubility parameter of the fragrance.

Selection of a fragrance 18 and a construction adhesive 35 that satisfy the above equation insures that fragrance 18 does not adversely interact with the construction adhesive 35 and thereby compromise the same. This insures that, even at high fragrance add on levels, the layers of the fragrance emitting patch 10a will remain secured adhered to one another and will not delaminate.

According the invention, the secondary layer 32 is a non-porous layer.

The porous secondary layer 32 may be a nonwoven material composed of only one type of fiber, such as polyester or polypropylene or it may include a mixture of more than one fiber. The secondary layer 32 may be composed of bi-component or conjugate fibers having a low melting point component and a high melting point component. The fibers may be selected from a variety of natural and synthetic materials such as nylon, polyester, rayon (in combination with other fibers), cotton, acrylic fiber and the like and combinations thereof. Bi-component fibers may be made up of a polyester layer and a polyethylene sheath. Using a fusible fabric increases the ease with which the secondary layer 32 may be mounted to an adjacent layer, e.g. the primary layer 12. According to another aspect of the invention, the secondary layer 32 is constructed from a microporous polymeric film material.

Referring to Figs. 4 and 5, a patch 10b includes according to a yet another embodiment of the invention, a porous primary layer 12 having a top surface 14 and a bottom surface 16. The primary layer 12 is provided with a fragrance represented by the numeral 18. The fragrance 18 may be applied to a top 14 or bottom 16 surface of the primary layer 12 or it may be infused into the primary layer 12 itself. The patch 10b further includes a secondary layer 32 having a top 34 and bottom surface 36. The bottom surface 36 of the secondary layer 32 is provided with the positioning adhesive 20 that permits a user to selectively apply the patch to a garment of clothing such as an undergarment or directly to the skin.

In the embodiment of the invention shown in Figs. 4 and 5 the primary layer 12 is secured to the secondary layer 32 in an adhesive free manner. For example, the primary layer 12 may be secured to the secondary layer by embossing using heat and pressure to fuse the primary layer 12 to the secondary layer 32. The primary layer 12 and secondary layer 32 may be constructed from nonwoven materials including heat fusible fibers to facilitate the bonding of the layers 12 and 32. In the embodiment shown in Fig. 4 and 5, the patch 10c includes a bonded area 40 around the periphery of the patch 10b.

The patches 10, 10a and 10b described herein preferably have a thickness in the range of between about 0.25 mm and about 2.0 mm.

### Example #1

A fragrance emitting patch according to the present invention may be constructed to include a 30 gsm primary layer made from a spunlace nonwoven material and a secondary non-porous layer made from 10 gsm polyethylene film. A sytrenic block copolymer construction adhesive having a solubility parameter $\delta_{ca}$ of 14.8 is applied to a bottom surface of the primary layer in an amount of 5 gsm to adhere the primary layer to the secondary layer. The bottom surface of the secondary layer is provided with a sytrenic block copolymers positioning adhesive having a solubility parameter $\delta_{pa}$ of 15. A fragrance consisting of Eugenol having a solubility parameter $\delta_f$ of 22.2 is applied to a top surface of the primary layer in an amount of 10 gsm. In this example, the absolute value difference of the solubility parameter of the construction adhesive and the fragrance is 7.4 and the absolute value difference of the solubility parameter of the positioning adhesive and the fragrance is 7.2. A ratio of the add on amount of construction adhesive relative to the add on amount of fragrance is 0.5.

### Example #2

Another fragrance emitting patch in accordance with the invention may be constructed in identical fashion to the patch described in Example #1 except that the secondary non-porous layer of example 1 is replaced with a 30 gsm layer spunlace nonwoven material.

### Claims

1. A fragrance emitting patch comprising:

   a primary porous layer having a top and a bottom surface;
   a secondary porous layer having a top and a bottom surface;
   a construction adhesive arranged between the primary layer and the secondary layer for securing the primary and secondary layers to one another;
   wherein the primary layer is provided with a fragrance in an amount within the range of 3 g/m$^2$ to 15 g/m$^2$; and
   wherein the absolute difference of the Hildebrand solubility parameter of the construction adhesive and the Hildebrand solubility parameter of the fragrance is greater than 1.5.

2. The fragrance emitting patch according to claim 1, further comprising a positioning adhesive arranged on the bottom surface of the secondary layer.

3. The fragrance emitting patch according to claim 2, wherein an absolute difference of a Hildebrand solubility parameter of the positioning adhesive and the Hildebrand solubility parameter of the fragrance is greater than 1.5.

4. A fragrance emitting patch comprising:

   a primary porous layer having a top and a bottom surface;
   a secondary porous layer having a top and a bottom surface;
   a positioning adhesive deposited on the bottom surface of the secondary layer;
   a construction adhesive arranged between the primary layer and the secondary layer for securing the primary and secondary layers to one another;
   wherein at least one of the first and second layers is provided with a fragrance in an amount within the range of 3 g/m$^2$ to 15 g/m$^2$; and
   wherein the absolute difference of the Hildebrand solubility parameter of the construction adhesive and the Hildebrand solubility parameter of the fragrance is greater than 1.5.

5. The fragrance emitting patch according to claim 4, wherein an absolute difference of a Hildebrand solubility parameter of the positioning adhesive and a Hildebrand solubility parameter of the fragrance is greater than 1.5.

6. The fragrance emitting patch according to any preceding claim, wherein the primary layer is a nonwoven material.

7. The fragrance emitting patch according to any preceding claim, wherein the primary layer is a microporous film.

8. The fragrance emitting patch of any preceding claim wherein the construction adhesive is a linear or radial co-polymer structure having the formula $(A-B)_x$ wherein block A is a polyvinylarene block, block B is a poly(monoalkenyl) block and x is an integer greater than or equal to one that denotes the number of polymeric arms.

9. The fragrance emitting patch of any preceding claim wherein the positioning adhesive is a linear or radial co-polymer structure having the formula $(A-B)_x$ wherein block A is a polyvinylarene block, block B is a poly(monoalkenyl)block and x is an integer greater than or equal to one that denotes the number of polymeric arms.

10. The fragrance emitting patch of any preceding claim wherein the ratio of the amount of construction adhesive to amount of fragrance is between 0.333 to 1.67.

**Patentansprüche**

1. Flecken, der einen Duftstoff verströmt, Folgendes umfassend:

   eine primäre poröse Schicht mit einer oberen und einer unteren Oberfläche,
   eine sekundäre poröse Schicht mit einer oberen und einer unteren Oberfläche,
   einen Konstruktionsklebstoff, der zwischen der primären Schicht und der sekundären Schicht angeordnet ist, um die primäre und die sekundäre Schicht aneinander zu befestigen,
   wobei die primäre Schicht mit einem Duftstoff in einer Menge im Bereich von 3 g/m$^2$ bis 15 g/m$^2$ versehen ist und
   wobei die absolute Differenz zwischen Hildebrandtschem Löslichkeitsparameter des Konstruktionsklebstoffs und Hildebrandtschem Löslichkeitsparameter des Duftstoffes größer als 1,5 ist.

2. Flecken, der einen Duftstoff verströmt, nach Anspruch 1, ferner einen Positionierungsklebstoff umfassend, der auf der unteren Oberfläche der sekundären Schicht angeordnet ist.

3. Flecken, der einen Duftstoff verströmt, nach Anspruch 2, wobei eine absolute Differenz zwischen Hildebrandtschem Löslichkeitsparameter des Positionierungsklebstoffs und Hildebrandtschem Löslichkeitsparameter des Duftstoffes größer als 1,5 ist.

**EP 2 039 371 B1**

4. Flecken, der einen Duftstoff verströmt, Folgendes umfassend:

eine primäre poröse Schicht mit einer oberen und einer unteren Oberfläche,
eine sekundäre poröse Schicht mit einer oberen und einer unteren Oberfläche,
einen Positionierungsklebstoff, der auf der unteren Oberfläche der sekundären Schicht abgeschieden ist,
einen Konstruktionsklebstoff, der zwischen der primären Schicht und der sekundären Schicht angeordnet ist, um die primäre und die sekundäre Schicht aneinander zu befestigen,
wobei mindestens entweder die primäre Schicht und/oder die sekundäre Schicht mit einem Duftstoff in einer Menge im Bereich von 3 g/m$^2$ bis 15 g/m$^2$ versehen ist und
wobei die absolute Differenz zwischen Hildebrandtschem Löslichkeitsparameter des Konstruktionsklebstoffs und Hildebrandtschem Löslichkeitsparameter des Duftstoffes größer als 1,5 ist.

5. Flecken, der einen Duftstoff verströmt, nach Anspruch 4, wobei eine absolute Differenz zwischen Hildebrandtschem Löslichkeitsparameter des Positionierungsklebstoffs und Hildebrandtschem Löslichkeitsparameter des Duftstoffes größer als 1,5 ist.

6. Flecken, der einen Duftstoff verströmt, nach einem der vorhergehenden Ansprüche, wobei die primäre Schicht aus einem Vliesmaterial besteht.

7. Flecken, der einen Duftstoff verströmt, nach einem der vorhergehenden Ansprüche, wobei die primäre Schicht eine mikroporöse Folie ist.

8. Flecken, der einen Duftstoff verströmt, nach einem der vorhergehenden Ansprüche, wobei der Konstruktionsklebstoff eine lineare oder radiale Copolymer-Struktur mit der Formel (A-B)$_x$ ist, wobei Block A ein Polyvinylaren-Block ist, Block B ein Poly(monoalkenyl)-Block ist und x eine Ganzzahl ist, die größer als eine Zahl oder gleich einer Zahl ist, die die Anzahl von Polymerarmen angibt.

9. Flecken, der einen Duftstoff verströmt, nach einem der vorhergehenden Ansprüche, wobei der Positionierungsklebstoff eine lineare oder radiale Copolymer-Struktur mit der Formel (A-B)$_x$ ist, wobei Block A ein Polyvinylaren-Block ist, Block B ein Poly(monoalkenyl)-Block ist und x eine Ganzzahl ist, die größer als eine Zahl oder gleich einer Zahl ist, die die Anzahl von Polymerarmen angibt.

10. Flecken, der einen Duftstoff verströmt, nach einem der vorhergehenden Ansprüche, wobei das Verhältnis der Menge an Konstruktionsklebstoff zur Menge an Duftstoff zwischen 0,333 und 1,67 liegt.

**Revendications**

1. Timbre émettant un parfum comprenant :

une couche poreuse primaire comportant une surface supérieure et une surface inférieure ;
une couche poreuse secondaire comportant une surface supérieure et une surface inférieure ;
un adhésif de construction disposé entre la couche primaire et la couche secondaire pour fixer la couche primaire et la couche secondaire l'une à l'autre ;
la couche primaire étant doté d'un parfum à une teneur comprise entre 3 g/m$^2$ et 15 g/m$^2$ ; et
la différence absolue d'indice de solubilité de Hildebrand entre l'adhésif de construction et le parfum étant supérieure à 1,5.

2. Timbre émettant un parfum conforme à la revendication 1, comprenant en outre un adhésif de positionnement disposé sur la surface inférieure de la couche secondaire.

3. Timbre émettant un parfum conforme à la revendication 2, où la différence absolue d'indice de solubilité de Hildebrand entre l'adhésif de positionnement et le parfum est supérieure à 1,5.

4. Timbre émettant un parfum comprenant :

une couche poreuse primaire comportant une surface supérieure et une surface inférieure ;
une couche poreuse secondaire comportant une surface supérieure et une surface inférieure ;

8

un adhésif de positionnement déposé sur la surface inférieure de la couche secondaire ;

un adhésif de construction disposé entre la couche primaire et la couche secondaire pour fixer la couche primaire et la couche secondaire l'une à l'autre ;

où au moins l'une des couches primaire et secondaire est dotée d'un parfum à une teneur comprise entre 3 $g/m^2$ et 15 $g/m^2$ ; et

la différence absolue d'indice de solubilité de Hildebrand entre l'adhésif de construction et le parfum étant supérieure à 1,5.

5. Timbre émettant un parfum conforme à la revendication 4, où la différence absolue d'indice de solubilité de Hildebrand entre l'adhésif de positionnement et le parfum est supérieure à 1,5.

6. Timbre émettant un parfum conforme à l'une quelconque des revendications précédentes, où la couche primaire est un matériau intissé.

7. Timbre émettant un parfum conforme à l'une quelconque des revendications précédentes, où la couche primaire est une pellicule microporeuse.

8. Timbre émettant un parfum conforme à l'une quelconque des revendications précédentes, où l'adhésif de construction est une structure copolymère linéaire ou radiale de formule $(A-B)_x$, où le bloc A est un bloc polyvinylarène, le bloc B est un bloc poly(monoalcényle) et x représente un entier supérieur ou égal à un entier représentant le nombre de bras polymères.

9. Timbre émettant un parfum conforme à l'une quelconque des revendications précédentes, où l'adhésif de positionnement est une structure copolymère linéaire ou radiale de formule $(A-B)_x$, où le bloc A est un bloc polyvinylarène, le bloc B est un bloc poly(monoalcényle) et x représente un entier supérieur ou égal à un entier représentant le nombre de bras polymères.

10. Timbre émettant un parfum conforme à l'une quelconque des revendications précédentes, où le rapport de la quantité de l'adhésif de construction sur la quantité de parfum est compris entre 0,333 et 1,67.

EP 2 039 371 B1

Fig. 1

Fig. 2

Fig. 3

10

Fig. 4

Fragrance

Fig. 5

Fig. 6

Fragrance

Fig. 7

**EP 2 039 371 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5399404 A
- US 20060251609 A